Europäisches Patentamt

European Patent Office

Office européen des brevets

(19)

(11) Veröffentlichungsnummer: **0 145 884**
A2

(12) # EUROPÄISCHE PATENTANMELDUNG

(21) Anmeldenummer: **84112383.9**

(22) Anmeldetag: **13.10.84**

(51) Int. Cl.⁴: **B 29 C 67/14**, B 32 B 27/32,
B 32 B 31/12
// B29K105/14

(30) Priorität: **03.12.83 DE 3343859**

(43) Veröffentlichungstag der Anmeldung: **26.06.85**
**Patentblatt 85/26**

(84) Benannte Vertragsstaaten: **AT BE CH DE FR GB IT LI NL
SE**

(71) Anmelder: **Felten & Guilleaume Energietechnik GmbH,
Schanzenstrasse 24-30 Postfach 80 50 01,
D-5000 Köln 80 (DE)**

(72) Erfinder: **Levacher, Friedrich Karl, Dr.,
Medardusstrasse 24, D-5024 P. Brauweiler (DE)**
Erfinder: **Federmann, Helmut, Dr., Holunderweg 17,
D-5060 Bergisch Gladbach (DE)**
Erfinder: **Dawihl, Walther, Prof.Dr.-Ing, Rassweilerweg 3,
D-6688 Illingen (DE)**

(54) **Verfahren zur Herstellung von Bauteilen aus unidirektionalen, kristallinen Kunststoffen.**

(57) Es wird ein Verfahren zur Herstellung von Bauteilen
aus unidirektionalen, kristallinen (uk-) Kunststoffen angegeben, die nicht nur als Ersatz von Bauteilen aus Verbundwerkstoffen dienen, sondern auch bei gleicher mechanischer Festigkeit ein geringeres Volumen einnehmen. Es
besteht im wesentlichen darin, daß durch ein spezielles
Anordnen, Formen und Miteinander-Verbinden von uk-
Kunststoffolien, das Bauteil nur noch aus einem einzigen
Werkstoff, der aus definierten Lagen kristalliner und amorpher Molekülverbände gebildet wird, besteht. Die Hauptanwendungsfälle zur Herstellung von Dünnfilmträgern für Magnetbänder und fotografische Filme und von in der Endoprothetik benutzten Ersatzteilen für beschädigte Körperteile
werden angegeben.

Verfahren zur Herstellung von Bauteilen aus unidirektionalen, kristallinen Kunststoffen

---

Die Erfindung betrifft ein Verfahren zur Herstellung von Bauteilen aus ultraverstreckten, unidirektionalen, kristallinen Kunststoffen (uk-Kunststoffen).

Es sind neuere Untersuchungen über faserartige Kristallstrukturen von Kunststoffen bekannt, die im Gegensatz zu den bisherigen amorphen und teilkristallinen Kunststoffen eine hohe Zugfestigkeit und einen hohen E-Modul aufweisen, siehe Abhandlung von J. Petermann "Eigenverstärkung von Kunststoffen" im Buch "Verbundwerkstoffe und Werkstoffverbunde in der Kunststofftechnik", VDI-Verlag Düsseldorf (1982) S. 83-100. Die theoretisch erwarteten und experimentell erreichten Zugfestigkeitswerte liegen beachtlich über denen der meisten in der Praxis verwendeten Verstärkungsfasern, wie Glas, Kevlar[R] sowie Nylon[R], und sie reichen an die der Kohlenstoffasern heran. Mit der Entwicklung praxisgerechter Verfahren zur Herstellung von uk-Kunststoffen, insbesondere uk-Polyäthylen (uk-PE), wurde begonnen. Damit rückt ihre Anwendung, insbesondere als Ersatz für Produkte aus Verbundwerkstoffen, in greifbare Nähe.

Verbundwerkstoffe etwa auf der Basis von GFK, SFK und CFK, die zur Zeit vorwiegend eingesetzt werden, sind dadurch gekennzeichnet, daß die Verstärkungsfasern aus Glas, Aramid[R] oder Kohle in eine Kunststoffmatrix eingebettet sind, die rund 40 %
des Volumens des Verbundbauteils beansprucht und zu dessen
Festigkeit nur untergeordnet beiträgt.

Der Erfindung liegt daher die Aufgabe zugrunde, ein Herstellungsverfahren für Bauteile aus uk-Kunststoffen anzugeben, die
nicht nur als Ersatz von Bauteilen aus Verbundwerkstoffen dienen, sondern auch bei gleicher mechanischer Festigkeit ein
geringeres Volumen einnehmen. Ferner sollen die wichtigsten
Anwendungsfälle angegeben werden.

Diese Aufgabe wird erfindungsgemäß durch die kennzeichnenden
Merkmale des Anspruchs 1 gelöst. Die Erfindung besteht im
wesentlichen darin, daß durch das spezielle Anordnen, Formen
und Miteinander-Verbinden der uk-Kunststoffolien das Bauteil
nur noch aus einem einzigen Werkstoff, der aus definierten
Lagen kristalliner und amorpher Molekülverbände gebildet wird,
besteht. Die bisherige Reaktionsharzmatrix entfällt.

Solche Bauteile haben bei gleicher mechanischer Festigkeit
eine geringere Wanddicke und ein niedrigeres spezifisches
Gewicht als die entsprechenden bisherigen Verbundbauteile.

Die Unteransprüche betreffen weitere Ausgestaltungen der Erfindung, so der Einsatz von Folien aus uk-Polyäthylen und die Ausbildung von Bändern oder Strängen (Ansprüche 2 und 3) und die
Anwendung des Verfahrens zur Herstellung von Dünnfilmträgern
für Magnetbänder und fotografische Filme (Anspruch 4) und von
in der Endoprothetik benutzten Ersatzteilen für beschädigte
Körperteile, insbesondere von Gelenkpfannen (Ansprüche 5 und 6).

Bei allen Anwendungen geht die Erfindung davon aus, daß bei
einer Folie aus faserkristallinen Kunststoffen die faserartig
unidirektional ausgerichteten Molekülketten der Folie im amorphen Kunststoff eingebettet sind. Die Schmelztemperatur des
amorphen uk-PE beträgt für die Kunststoffmatrix etwa 130 $^{o}$C,
wogegen die Rekombinationstemperatur der unidirektionalen
Faserkristalle bei 148 $^{o}$C liegt.

Welchen Vorteil das geringere spezifische Gewicht des uk-PE
bietet, erkennt man daran, daß gegenüber dem bisherigen Stand
der Technik Magnetbänder aus uk-PE-Trägerfolien eine 5- bis
10-mal kleinere Dicke haben. Sie gestatten daher unter sonst
gleichen Bedingungen, die Speicherkapazität um das 5- bis
10-fache zu erhöhen. Bei fotografischen Filmen wäre es möglich,
die Speicherkapazität um etwa das 15-fache zu steigern.

Drei Anwendungsbeispiele der Erfindung sind in der Zeichnung
schematisch dargestellt und werden im folgenden näher beschrieben. Es zeigen:
Fig. 1 einen Druckbehälter aus einem Verbund von unidirektionalem, kristallinem Polyäthylen (uk-PE),
Fig. 2 einen gewickelten Strang aus uk-PE und
Fig. 3 eine Gelenkpfanne mit einer Einlage aus uk-PE.

Bezeichnet sind mit:
1 Druckbehälter-Außenwand aus uk-PE-Folie
2 Stränge aus uk-PE in Umfangsrichtung für Radialkraftaufnahme
3   "    "    "   in Längsrichtung für Achsialkraftaufnahme
4 Strangseele aus längslaufender, eingerollter uk-PE-Folie
5 Seelenumwicklung aus uk-PE-Bändern
6 uk-PE-Einlage einer Genlenkpfanne
7 Pfannengrundkörper aus Aluminiumoxid oder PE.

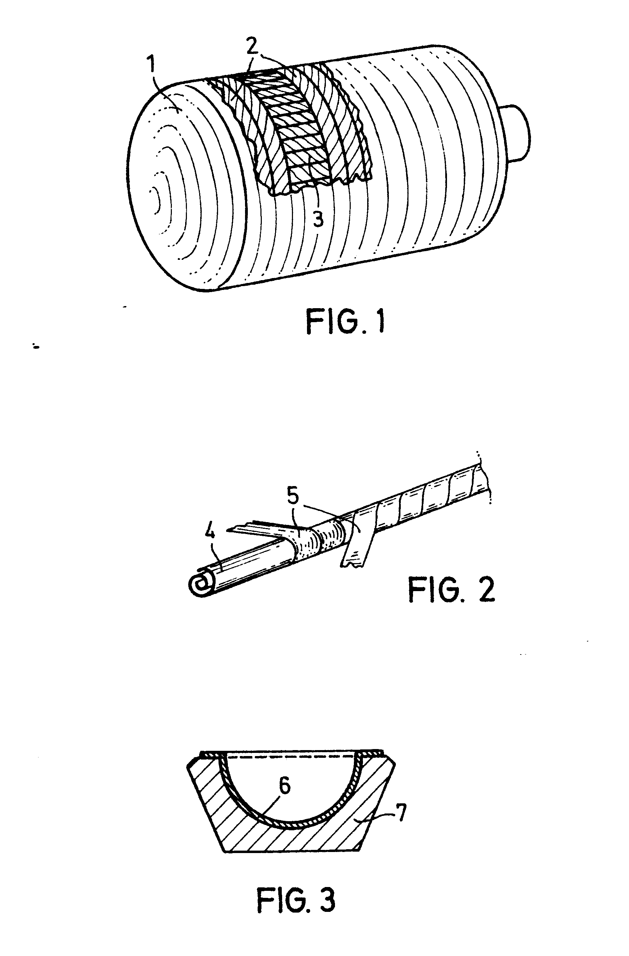

Fig. 1 zeigt den Aufbau eines Druckbehälters aus einem uk-PE-
Verbund. Die Außenwand 1 besteht aus uk-PE-Folie. Darunter ist
eine Lage in Umfangsrichtung verlaufender uk-PE-Stränge 2 angeordnet, welche die Radialkräfte aufnehmen. Darunter ist eine
Lage in Achsrichtung des Druckbehälters verlaufender uk-PE-
Stränge 3 angeordnet, welche die Achsialkräfte aufnehmen. Weitere uk-PE-Stranglagen folgen, bis die unterste Lage den rohrförmigen Behälter des unter Druck stehenden Mediums umgibt.

Fig. 2 zeigt den Aufbau eines gewickelten Stranges aus uk-PE.
Die Strangseele 4 wird aus längslaufender, eingerollter uk-PE-
Folie gebildet. darüber befinden sich zwei Lagen einer Umwick-

Fl 4712                          4                  0145884

lung aus uk-PE-Bändern 5, deren Wickelrichtungen sich kreuzen.

Fig. 3 zeigt eine Gelenkpfanne mit einer uk-PE-Einlage. In der
Endoprothetik werden beim Ersatz beschädigter oder ausgefallener Körperteile, wie Sehnen, Bänder, Ventilklappen, Adern und
Genlenkteile, Werkstoffe verwendet, von denen Verträglichkeit
unter Biobedingungen, Festigkeitsverhalten, Widerstand gegen
thermoplastische Verformungen und in manchen Fällen auch Verschleißfestigkeit verlangt werden. Als Werkstoffe kommen eisenfreie Metallegierungen und keramische Werkstoffe, insbesondere
höchstreines Aluminiumoxid im Sinterzustand, in Betracht. Bei
den Kunstharzen ist neben den Anforderungen mechanischer Art
besonders ihre Verträglichkeit gegenüber Körperflüssigkeiten
zu bachten. Dabei haben sich Polyparaffine, insbesondere PE,
wegen ihrer guten biologischen Verträglichkeit in der Praxis
eingeführt.

Es liegen längjährige, gute Erfahrungen beim Einsatz von Hüftgelenkendoprothesen mit einer Gelenkkugel aus Aluminiumoxid
und Pfannen aus dem gleichen Werkstoff vor. Allerdings können
bei einer Beanspruchung durch Springen oder Fallen Aluminiumoxidteilchen abbrechen, die das Reibungsverhältnis zwischen
Kugel und Pfanne beeinträchtigen. Pfannen aus PE in Kombination mit einer Kugel aus Aluminiumoxid haben zwar eine geringere Stoßempfindlichkeit, aber auch eine geringere Verschleißfestigkeit, die vom PE herrührt. Versuche, die Eigenschaften
des PE durch den Einbau von Graphit oder anderen anorganischen
Pulvern zu verbessern, haben jedoch nicht zu befriedigenden
Ergebnissen geführt.

Hier wird nun bei der in der Fig. 3 gezeigten Anordnung einer
Einlage 6 aus uk-PE in der Innenform des Pfannengrundkörpers 7
aus Aluminiumoxid oder PE, oder bei der Ausbildung der ganzen
Pfanne aus uk-PE eine wesentliche Verbesserung erzielt. Die
Verschleißfestigkeit nähert sich der des Aluminiumoxid an. Die
biochemische Verträglichkeit von uk-PE ist gegenüber der von
PE nicht beeinträchtigt.

Fl 4712        5        0145884

Während die Formgebung bei der Gelenkpfanne aus uk-PE durch
Warmpressung erfolgt, stellt man Adern aus uk-PE-Folien in
einem für Schläuche bekannten Wickelverfahren her. Dabei bleibt
trotz der hohen Steifigkeit des uk-PE eine für die Praxis ausreichende Verformbarkeit erhalten.

0145884

F1 4712                         1                    23.11.83

1. Verfahren zur Herstellung von Bauteilen aus ultraverstreckten, unidirektionalen, kristallinen Kunststoffen (uk-Kunststoffen), d a d u r c h   g e k e n n z e i c h n e t,
   daß
   a) Folien aus einem uk-Kunststoff so zu einem Bauteil angeordnet, geformt und miteinander verbunden werden, daß
      die Faserkristalle in Richtung der aufzunehmenden Zug-
      oder Druckkräfte liegen, wobei
   b) eine Vielzahl von Folien, gegebenenfalls mit verschie-
      denen Orientierungsrichtungen der Faserkristalle auf-
      einandergelegt und dadurch kraftschlüssig miteinander
      verbunden werden, daß sie auf die Schmelztemperatur der
      amorphen Kunststoffmatrix erhitzt und unter Druck miteinander verschweißt und wieder abgekühlt werden,und
   c) die Formgebung bei flachen Teilen durch ein Warmpreßverfahren, bei runden Teilen durch ein Wickelverfahren
      erfolgt, wobei durch Wickeln der einzelnen Lagen unter
      verschiedenen Winkeln ein Ausgleich in den mechanischen
      Eigenschaften in Abhängigkeit von der Beanspruchungsrichtung erreicht wird.

2. Verfahren nach Anspruch 1, d a d u r c h   g e k e n n -
   z e i c h n e t,  daß Folien aus uk-Polyparaffinen, vorzugsweise uk-Polyäthylen (uk-PE), mit einer Foliendicke
   zwischen 1 und 10 $\mu$m eingesetzt werden, und daß die uk-
   PE-Folien bei Temperaturen zwischen 130 und 148 $^{\circ}$C miteinander verschweißt werden.

Fl 4712                    2                    0145884

3. Verfahren nach Anspruch 1 oder 2, d a d u r c h   g e -
   k e n n z e i c h n e t, daß die Folien aus uk-Kunststoffen zu Bändern oder Strängen kraftschlüssig miteinander verbunden werden, wobei eine Anzahl der Bänder zu
   Strängen gewickelt oder verseilt wird.

4. Anwendung des Verfahrens nach einem der Ansprüche 1 oder 2
   für die Herstellung von Magnetbändern zum Aufzeichnen von
   Bild-, Ton- und Dateninformationen oder von Trägern für
   fotografische Filme.

5. Anwendung des Verfahrens nach einem der Ansprüche 1 bis 3
   für die Herstellung von in der Endoprothetik benutzter
   Ersatzteile für beschädigte oder ausgefallene Körperteile,
   wie Sehnen, Bänder, Ventilklappen, Adern oder Gelenkteile
   sowie auch Fäden zum Vernähen von Wunden.

6. Anwendung des Verfahrens nach Anspruch 5 für die Herstellung einer Gelenkpfanne von etwa 20 mm Höhe, 35 mm Innendurchmesser der halbkugelförmigen Innenform und 50 mm
   Außendurchmesser, bei der in der Innenform des Pfannengrundkörpers (7) eine Einlage (6) aus uk-PE von etwa 1 mm
   Dicke angebracht wird oder die ganze Pfanne aus uk-PE gebildet wird, wobei die Formgebung durch Einlegen eines
   Filmpaketes in eine zweiteilige Stahlform, Erwärmen und
   Einpressen des kugelförmigen Oberstempels erfolgt.

FI 4712

0145884

FIG. 1

FIG. 2

FIG. 3